# EUROPÄISCHE PATENTSCHRIFT

(11) **EP 0 752 422 B1**
(45) Veröffentlichungstag und Bekanntmachung des Hinweises auf die Patenterteilung: **23.07.2003**
(21) Anmeldenummer: 96110355.3
(22) Anmeldetag: 27.06.1996
(51) Int. Cl.: C07D 233/58, C07D 235/06, C07D 249/08

(54) **Verfahren zur Herstellung von N-Alkenyl-azolen**
Process for the preparation of N-alkenyl-azoles
Procédé pour la préparation de N-alkényle-azoles

(30) Priorität: 06.07.1995 DE 19524618
(43) Veröffentlichungstag der Anmeldung: 08.01.1997
(73) Patentinhaber: BASF AKTIENGESELLSCHAFT, 67056 Ludwigshafen (DE)
(72) Erfinder: Henkelmann, Jochem, Dr., 68165 Mannheim (DE); Heider, Marc, Dr., 67433 Neustadt (DE); Rühl, Thomas, Dr., 67227 Frankenthal (DE)

(56) Entgegenhaltungen:
- EP-A- 0 697 398
- DE-A- 2 725 379
- US-A- 5 023 375
- DATABASE WPI Week 7223 Derwent Publications Ltd., London, GB; AN 72-37610T XP002014074 & JP-B-47 020 011 (TOA GOSEI CHEMICAL INDUSTRY CO.)
- CHEMISTRY OF HETEROCYCLIC COMPOUNDS, Bd. 25, Nr. 6, Oktober 1989, Seite 714 XP000602679 LEBEDEVA N.P. & KALAUS I.V.: "Formation of N-vinyl derivatives of 1,2,4-triazole" & KHIMIYA GETEROTSIKLICHESKIKH SOEDINENII, Nr. 6, April 1989, Seite 856

## Beschreibung

Die vorliegende Erfindung betrifft ein neues Verfahren zur Herstellung von N-Alkenyl-azolen durch Umsetzung von Azolen mit einem Carbonsäurealkenylester bei erhöhten Temperaturen in Gegenwart einer Base und anschließend unter Zusatz eines quartären Ammonium- oder Phosphoniumsalzes.

Aus Liebigs Annalen der Chemie, Band 601, Seiten 133 ff. (1956) ist die Herstellung von N-Vinylimidazolen durch Umsetzung von Imidazolen mit Acetylen unter Druck in Gegenwart von Basen bekannt.

Der Umgang mit Acetylen erfordert einen großen technischen Aufwand im Hinblick auf die Acetylenverfügbarkeit und den sicheren Umgang mit Acetylen. Besonders ist der sicheren Reaktionsführung Aufmerksamkeit zu schenken, da der für die Umsetzung von Imidazolen mit Acetylen verwendete Katalysator-Kaliumhydroxid einen Acetylenzerfall im Reaktionssystem auslösen kann. Dies haben jüngste Untersuchungen von Schildberg et al. gezeigt (Chem. Ing. Tech. 66 (1994) 1389 bis 1392).

In der JP-Auslegeschrift 47020011-B ist die Umsetzung von Lactamen mit Vinylestern in Gegenwart eines Quecksilbersalzes und einer Säure zu N-Vinyllactamen beschrieben.

In US 5,023,375 ist die Synthese von Ethyliden-bis-amid-Derivaten druch Umsetzung von Vinylacetat mit primären Carbonsäureamiden in Gegenwart eines Palladium-, Platin- oder Quecksilbersalzes und eines Alkohols beschrieben.

Die DE-Offenlegungsschrift 27 25 379 offenbart die Herstellung von N-Vinylamiden und N-Vinylimiden durch Umsetzung der entsprechenden Amide und Imide mit Vinylestern in Gegenwart eines Palladiumsalzes.

EP-A 0 697 398 lehrt die Herstellung von N-Alkenylcarbonsäureamiden durch Umsetzung von Carbonsäurealkenylestern mit Carbonsäureamiden in Gegenwart einer Base.

N.P. Lebedeva et al., Chemistry of Heterocyclic Compounds, Band 25, Nummer 6, 1989, Seite 714 lehrt die Herstellung von N-Vinyltriazol-Derivaten durch Umsetzung von Triazol-Derivaten mit Vinylacetat in Gegenwart von Quecksilberacetat-Bortrifluorid-Etherat.

Der vorliegenden Erfindung lag daher die Aufgabe zugrunde, den zuvor genannten Nachteilen abzuhelfen.

Demgemäß wurde ein neues und verbessertes Verfahren zur Herstellung von N-Alkenyl-azole der allgemeinen Formel I 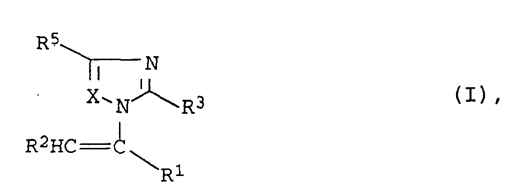 in der
- X: CH oder Stickstoff,
- R¹,R²: Wasserstoff oder C₁- bis C₈-Alkyl,

- R³,R⁵: Wasserstoff, C₁- bis C₄₀-Alkyl, C₂- bis C₄₀-Alkenyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl, durch C₁- bis C₈-Alkyl, C₁- bis C₈-Alkoxy, Halogen ein- bis fünffach substituiertes Aryl, C₇- bis C₂₀-Alkylaryl, C₇- bis C₂₀-Aralkyl
bedeuten, durch Umsetzung von Azolen der allgemeinen Formel II 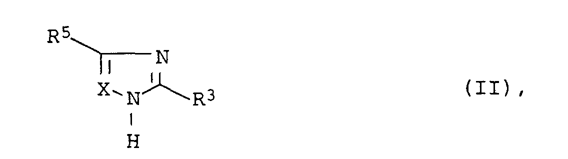 in der X, R³ und R⁵ die oben genannten Bedeutungen haben, mit einem Carbonsäurealkenylester der allgemeinen Formel III 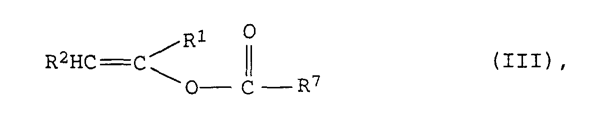 in der R¹ und R² die oben genannten Bedeutungen haben und R⁷ Wasserstoff, C₁- bis C₄₀-Alkyl, C₃- bis C₂₀-Cycloalkyl, C₄- bis C₂₀-Alkyl-cycloalkyl, Aryl, C₇- bis C₂₀-Alkylaryl, C₇- bis C₂₀-Aralkyl, durch C₁- bis C₈-Alkyl ein- bis dreifach substituiertes Aryl oder C₇- bis C₂₀-Aralkyl bedeutet, bei Temperaturen von 0 bis 180°C und Drücken von 0,01 bis 10 bar gefunden, welches dadurch gekennzeichnet ist, daß man die Umsetzung in Gegenwart einer Base und eines quartären Ammonium- oder Phosphoniumsalzes durchführt.

Das erfindungsgemäße Verfahren läßt sich wie folgt durchführen:

Die Azole II und die Carbonsäurealkenylester III können kontinuierlich oder diskontinuierlich in Anwesenheit oder bevorzugt in Abwesenheit des Zusatzes eines inerten Lösungsmittels bei Temperaturen von 0 bis 180°C, bevorzugt 40 bis 150°C, besonders bevorzugt 50 bis 120°C und Drücken von 0,01 bis 10 bar, bevorzugt 0,1 bis 2 bar, besonders bevorzugt Atmosphärendruck (Normaldruck) in Gegenwart einer Base und eines quartären Ammoniumoder Phosphoniumsalzes umgesetzt werden, wobei die Edukte in beliebiger Reihenfolge zusammengegeben werden können. So können die Ausgangsverbindungen II und III, die Base und das quartäre Ammonium- oder Phosphoniumsalz z.B. in einen Rührkessel gegeben und darin umgesetzt werden. Es ist auch möglich, die Ausgangsverbindungen II und III, die Base und das quartäre Ammonium- oder Phosphoniumsalz in einem Rohrreaktor z.B. in Rieseloder Sumpffahrweise umzusetzen. Es hat sich als vorteilhaft erwiesen, die Reaktion in einem Strahldüsenreaktor vorzunehmen.

Als Basen eignen sich anorganische oder organische Basen, vorzugsweise Brönsted-Basen, z.B. Carbonate und Hydrogencarbonate der Alkali- und Erdalkalimetalle wie Natriumcarbonat, Kaliumcarbonat und Natriumhydrogencarbonat, quartäre Ammoniumcarbonate wie Tetramethylammoniumcarbonat, Amide wie Alkalimetallamide, beispielsweise Natriumamid und Kaliumamid, Hydroxide wie Alkalimetallhydroxide, beispielsweise Lithiumhydroxid, Natriumhydroxid und Kaliumhydroxid, Alkalimetallcarboxylate wie Natriumacetat, Alkoholate wie Alkalimetallalkoholate, beispielsweise Natriummethanolat, Natriumethanolat, Kaliummethanolat und Kalium-tert.butanolat. Kaliumhydroxid kann auch zusammen mit Kronenethern wie 18-Krone-6 verwendet werden.

Als Basen eignen sich weiterhin Ammoniak, primäre, sekundäre und tertiäre Amine, bevorzugt tertiäre Amine. Die Amine können aliphatische oder aromatische Reste tragen, beispielsweise Trialkylamine wie Trioctylamin, Ethyldiisopropylamin, Diethylisopropylamin, Dimethylcyclohexylamin, Triethylamin, außerdem cyclische Amine wie 2,2,6,6-Tetramethylpiperidin, 1,4-Diazabicyclo[2.2.2]octan, 1,8-Diazabicyclo-[5.4.0]-undec-7-en, aliphatische und aromatische Reste tragende Amine wie 1,8-Bis(dimethylamino)-naphthalin und 4-Dimethylaminopyridin und heterocyclische Amine wie N-Alkylimidazole und N-Arylimidazole. Weiterhin kommen Amide wie Dialkylcarbonsäureamide, z. B. Dibutylformamid, in Betracht. Das erfindungsgemäße Verfahren läßt sich auch in Gegenwart von basischen Ionenaustauschern, die in der Regel aus sulfonierten Styrol-Divinylbenzol-Copolymerisaten bestehen wie Amberlite®, Lewatit® und Puralit®, und in Gegenwart von basischen Zeolithen wie Hydrotalcit durchführen.

Als quartäre Ammonium- und Phosphoniumsalze eignen sich beispielsweise Tetramethylphosphoniumchlorid, Tetramethylammoniumchlorid, Tetraethylphosphoniumchlorid, Tetraethylammoniumchlorid, Tetramethylphosphoniumbromid, Tetramethylammoniumbromid, Tetraethylphosphoniumbromid, Tetraethylammoniumbromid, N,N-Dimethylpiperidiniumchlorid und Trimethylbenzylammoniumchlorid, bevorzugt Tetraethylammoniumbromid, besonders bevorzugt N,N'-Dimethylpiperidiniumchlorid. Das Molverhältnis von Carbonsäurealkenylester III zum Azol II beträgt in der Regel 0,1:1 bis 10:1, bevorzugt 0,9:1 bis 5:1, besonders bevorzugt 1:1 bis 1,2:1.

Das Molverhältnis von Base zum Azol II beträgt in der Regel 0,1:1 bis 10:1, bevorzugt 0,2:1 bis 4:1, besonders bevorzugt 0,2:1 bis 2:1.

Das Molverhältnis von quartärem Ammonium- oder Phosphoniumsalz zum Azol II beträgt in der Regel 0,0001:1 bis 5:1, bevorzugt 0,1:1 bis 0,8:1, besonders bevorzugt 0,001:1 bis 0,4:1.

Als inerte Lösungsmittel eignen sich beispielsweise aprotische Lösungsmittel z.B. Ether wie Tetrahydrofuran, aromatische Kohlenwasserstoffe wie Toluol und Xylol, Ketone wie Aceton, weiterhin Acetonitril, Hexamethylphosphorsäuretriamid, Sulfolan, Dimethylsulfoxid, Harnstoffe wie N,N'-Dimethylethylen-, N,N'-Dimethylpropylenharnstoff und Tetrabutylharnstoff. Die Menge liegt in der Regel bei 5 bis 300 Gew.-%, bevorzugt 10 bis 100 Gew.-%, besonders bevorzugt 10 bis 30 Gew.-% bezogen auf den Gesamtansatz.

In der Regel ist die Reaktion nach 5 Minuten bis 8 Stunden beendet.

Das so erhaltene Reaktionsgemisch kann in an sich bekannter Weise aufgearbeitet werden. In der Regel wird das N-Alkenylazol I destillativ abgetrennt. Der Destillationssumpf kann zur Freisetzung der organischen Basen aus den bei der Reaktion anfallenden Salzen mit Laugen wie Natronlauge versetzt werden. Die beigesetzten Basen können anschließend extraktiv oder destillativ isoliert werden. Werden in der erfindungsgemäßen Umsetzung leichtflüchtige salzartige Verbindungen wie Formiate tertiärer Ammoniumverbindungen gebildet, können diese auch destillativ aufgearbeitet und in die entsprechenden Amine überführt werden. Die jeweils abgetrennten Basen können in die Reaktion zurückgeführt werden.

Die Substituenten R¹, R², R³, R⁵, R⁷ und das Zwischenglied X in den Verbindungen I, II und III haben folgende Bedeutungen:
X
   - CH,
   - Stickstoff,
R¹,R²,R³,R⁵,R⁷
   - Wasserstoff,
R¹,R²
   - C₁- bis C₈-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
R³,R⁵, R⁷
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkylcycloalkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl und 2-Phenylethyl,
R³,R⁵
   - C₁- bis C₄₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₄₀-Alkenyl, bevorzugt C₂- bis C₁₂-Alkenyl, besonders bevorzugt C₂- bis C₈-Alkenyl wie Vinyl und Propenyl
   - C₄- bis C₂₀-Cycloalkyl-alkyl, bevorzugt C₄- bis C₁₂-Cycloalkyl-alkyl, besonders bevorzugt C₅- bis C₁₀-Cycloalkylalkyl,
   - durch C₁- bis C₈-Alkyl, C₁- bis C₈-Alkoxy, Halogen ein- bis fünffach substituiertes Aryl, bevorzugt durch C₁- bis C₈-Alkyl, C₁- bis C₈-Alkoxy, Halogen ein- bis dreifach substituiertes Phenyl wie Tolyl und Anisyl,
   - durch C₁- bis C₈-Alkyl, C₁- bis C₈-Alkoxy, Halogen ein- bis fünffach substituiertes C₇- bis C₂₀-Alkylaryl, bevorzugt durch C₁- bis C₈-Alkyl, C₁- bis C₈-Alkoxy, Halogen ein- bis dreifach substituiertes C₇- bis C₁₆-Alkylphenyl,
   - durch C₁- bis C₈-Alkyl, C₁- bis C₈-Alkoxy, Halogen ein- bis fünffach substituiertes C₇- bis C₂₀-Aralkyl, bevorzugt durch C₁- bis C₈-Alkyl, C₁- bis C₈-Alkoxy, Halogen ein- bis dreifach substituiertes C₇- bis C₁₆-Phenalkyl,
R7
   - C₁- bis C₄₀-Alkyl, bevorzugt C₁- bis C₂₀-Alkyl, besonders bevorzugt Methyl, Ethyl, n-Propyl, iso-Propyl, n-Butyl, tert.-Butyl, n-Pentyl, n-Hexyl, n-Heptyl, n-Octyl, n-Decyl, n-Dodecyl und n-Octadecyl,
   - durch C₁- bis C₈-Alkyl ein- bis dreifach substituiertes Aryl, bevorzugt durch C₁- bis C₄-Alkyl ein- bis dreifach substituiertes Phenyl wie 2-Methylphenyl,
   - durch C₁- bis C₈-Alkyl ein- bis dreifach substituiertes C₇bis C₂₀-Aralkyl, bevorzugt durch C₁- bis C₄-Alkyl ein- bis dreifach substituiertes C7- bis C₁₆-Phenalkyl.

Als Carbonsäurealkenylester III seien bevorzugt genannt: Vinylformiat, Vinylacetat, Vinylpropionat, Vinylstearat (Stearinsäurevinylester oder Octadecansäurevinylester), Vinylpivalat (Pivalinsäurevinylester oder 2,2-Dimethylpropionsäurevinylester) und 4-tert.-Butylbenzoylvinylester.

Die Carbonsäurealkenylester III sind käuflich oder nach bekannten Methoden herstellbar, beispielsweise durch Addition von Carbonsäuren an Acetylen oder durch Acetoxilierung von Ethylen (Industrielle Organische Chemie, 2. Auflage, 1978, Verlag Chemie, Seite 217 bis 223).

Als Azole II seien bevorzugt genannt: Imidazol und 1,2,4-Triazol.

Auch diese Verbindungen sind käuflich oder nach bekannten Methoden erhältlich.

Die Verfahrensprodukte der Formel I sind gesuchte Zwischenprodukte. N-Alkenylazole, insbesondere die N-Alkenylimidazole können in bekannter Weise polymerisiert werden. Diese Polymere, insbesondere von N-Vinylimidazol und N-Vinyl-2-methyl-imidazol, dienen beispielsweise als Waschhilfsmittel.

### Beispiele

### Beispiele 1 bis 6

Das Azol II, der Carbonsäurealkenylester III und die Base wurden in den in Tabelle 1a angegebenen Mengen bei den dort genannten Temperaturen und 1 bar umgesetzt. Anschließend wurde ohne Isolierung die in Tabelle 1b angegebene Menge des quartären Ammoniumsalzes zugegeben und die Temperatur wie aus Tabelle 1b ersichtlich angehoben. Der Verlauf der Reaktion wurde gaschromatographisch verfolgt. Nach beendeter Reaktion erfolgte die Isolierung durch Destillation.

Die Einzelheiten sind Tabelle 1a und Tabelle 1b zu entnehmen:

**Tabelle 1a**

| Beispiel Nr.: | Azol II [mmol] | Ester III [mmol] | Base [mmol] | Temperatur [°C] |
|---|---|---|---|---|
| 1 | 300 mmol Imidazol | 360 mmol Vinylformiat | 300 mmol Triethylamin | 50 |
| 2 | 300 mmol Imidazol | 300 mmol Vinylacetat | 150 mmol Triethylamin | 60 |
| 3 | 300 mmol 2-Methyl-imidazol | 300 mmol Vinylacetat | 150 mmol Triethylamin | 60 |
| 4 ^{b)} | 300 mmol Benzimidazol | 500 mmol Vinylformiat | 500 mmol Dimethylcyclohexylamin | 60 |
| 5 | 300 mmol 4-Methyl-imidazol | 300 mmol Vinyl-propionat | 300 mmol Triethylamin | 90 |
| 6 ^{a)} | 1 mol 1,2,4-Triazol | 1,1 mol Vinylacetat | 0,5 mol 4-N,N-Dimethylaminopyridin | 80 |

| | | | | |
|---|---|---|---|---|
| ^{a)} Zugabe von 100 g THF | | | | |
| ^{b)} Vergleichsbeispiel | | | | |

**Tabelle 1b**

| Beispiel Nr.: | N,N-Dimethylpiperidiniumchlorid (quartäres Salz) [mmol] | Temperatur [°C] | Alkenyl-azol I | Ausbeute [%] |
|---|---|---|---|---|
| 1 | 0,33 | 140 | N-Vinylimidazol | 85 |
| 2 | 0,33 | 130 | N-Vinylimidazol | 92 |
| 3 | 0,5 | 140 | N-Vinyl-2-methyl-imidazol | 89 |
| 4^{d)} | 0,5 | 150 | N-Vinylbenzimidazol | 87 |
| 5 | 0,33 | 140 | N-Vinyl-4-methyl-imidazol | 91 |
| 6 | 1,5 | 150^{c)} | N-Vinyl-triazol | 87 |

| | | | | |
|---|---|---|---|---|
| ^{c)} nach Abdestillieren des Lösungsmittels THF | | | | |
| ^{d)} Vergleichsbeispiel | | | | |

## Patentansprüche

1. Verfahren zur Herstellung von N-Alkenyl-azole der allgemeinen Formel I 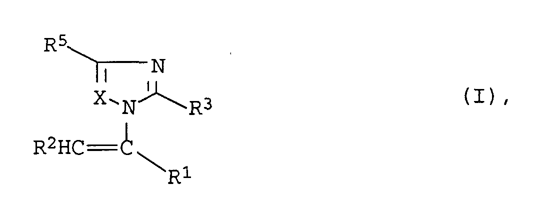 in der
X CH oder Stickstoff,
R¹,R² Wasserstoff oder C₁- bis C₈-Alkyl,
R³,R⁵ Wasserstoff, C₁- bis C₄₀-Alkyl, C₂- bis C₄₀-Alkenyl, C₃- bis C₂₀-Cycloalkyl, C₄- bis C₂₀-Alkyl-cycloalkyl, C₄- bis C₂₀-Cycloalkylalkyl, Aryl, C₇- bis C₂₀-Alkylaryl, C₇- bis C₂₀-Aralkyl, durch C₁- bis C₈-Alkyl, C₁- bis C₈-Alkoxy, Halogen ein- bis fünffach substituiertes Aryl, C₇- bis C₂₀-Alkylaryl, C₇- bis C₂₀-Aralkyl
bedeuten, durch Umsetzung von Azolen der allgemeinen Formel II 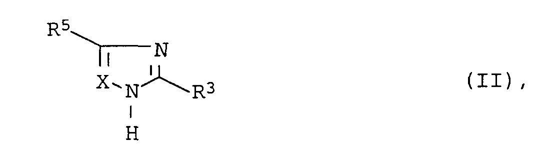 in der X, R³ und R⁵ die oben genannten Bedeutungen haben, mit einem Carbonsäurealkenylester der allgemeinen Formel III 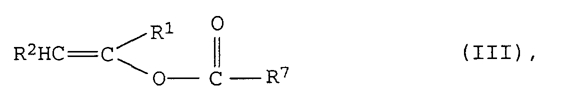 in der R¹ und R² die oben genannten Bedeutungen haben und R⁷ Wasserstoff, C₁- bis C₂₀-Alkyl, C₃- bis C₂₀-Cycloalkyl, C₄bis C₂₀-Alkyl-cycloalkyl, Aryl, C₇- bis C₂₀-Alkylaryl, C₇bis C₂₀-Aralkyl, durch C₁- bis C₈-Alkyl ein- bis dreifach substituiertes Aryl oder C₇- bis C₂₀-Aralkyl bedeutet, bei Temperaturen von 0 bis 180°C und Drücken von 0,01 bis 10 bar, **dadurch gekennzeichnet, daß** man die Umsetzung in Gegenwart einer Base und eines quartären Ammonium- oder Phosphoniumsalzes durchführt.

2. Verfahren zur Herstellung von N-Alkenyl-azolen nach Anspruch 1, **dadurch gekennzeichnet, daß** R³ und R⁵ für Wasserstoff oder C₁- bis C₂₀-Alkyl stehen.

3. Verfahren zur Herstellung von N-Alkenyl-azolen nach den Ansprüchen 1 bis 2, **dadurch gekennzeichnet, daß** R³ und R⁵ für Wasserstoff oder C₁- bis C₁₂-Alkyl stehen.

4. Verfahren zur Herstellung von N-Alkenyl-azolen nach den Ansprüchen 1 bis 3, **dadurch gekennzeichnet, daß** R³ und R⁵ für Wasserstoff stehen.

5. Verfahren zur Herstellung von N-Alkenyl-azolen nach den Ansprüchen 1 bis 4, **dadurch gekennzeichnet, daß** R¹ und R² für Wasserstoff stehen.

6. Verfahren zur Herstellung von N-Alkenyl-azolen nach den Ansprüchen 1 bis 5, **dadurch gekennzeichnet, daß** R⁷ für Wasserstoff, C₁- bis C₂₀-Alkyl, C₃- bis C₈-Cycloalkyl, Aryl, C₇bis C₂₀-Alkylaryl oder C₇- bis C₂₀-Aralkyl steht.

7. Verfahren zur Herstellung von N-Alkenyl-azolen nach den Ansprüchen 1 bis 6, **dadurch gekennzeichnet, daß** man die Umsetzung bei Temperaturen von 40 bis 150°C durchführt.

8. Verfahren zur Herstellung von N-Alkenyl-azolen nach den Ansprüchen 1 bis 7, **dadurch gekennzeichnet, daß** man die Umsetzung bei Temperaturen von 50 bis 120°C durchführt.

9. Verfahren zur Herstellung von N-Alkenyl-azolen nach den Ansprüchen 1 bis 8, **dadurch gekennzeichnet, daß** man die Umsetzung bei Drücken von 0,1 bis 2 bar durchführt.

10. Verfahren zur Herstellung von N-Alkenylazolen nach den Ansprüchen 1 bis 9, **dadurch gekennzeichnet, daß** man die Umsetzung bei Atmosphärendruck durchführt.

## Claims

1. A process for preparing N-alkenylazoles of the general formula I 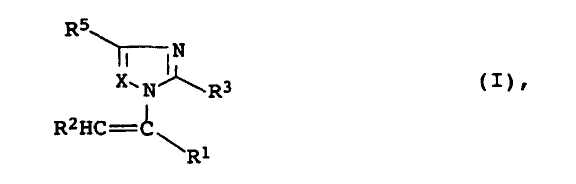 where
X is CH or nitrogen,
R¹ and R² are hydrogen or C₁- to C₈-alkyl,
R³ and R⁵ are hydrogen, C₁- to C₄₀-alkyl, C₂- to C₄₀-alkenyl, C₃- to C₂₀-cycloalkyl, C₄- to C₂₀-alkylcycloalkyl, C₄- to C₂₀-cycloalkylalkyl, aryl, C₇- to C₂₀-alkylaryl or C₇- to C₂₀-aralkyl, or aryl, C₇- to C₂₀-alkylaryl or C₇- to C₂₀-aralkyl which are mono- to pentasubstituted by C₁- to C₈-alkyl, C₁- to C₈-alkoxy or halogen,
by reaction of azoles of the general formula II 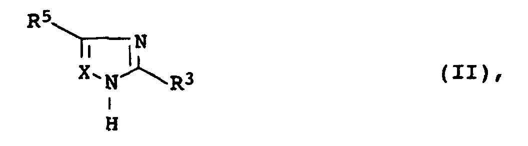 where X, R³ and R⁵ have the abovementioned meanings, with an alkenyl carboxylate of the general formula III 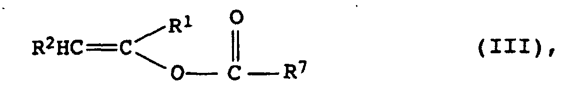 where R¹ and R² have the abovementioned meanings and R⁷ is hydrogen, C₁- to C₄₀-alkyl, C₃- to C₂₀-cycloalkyl, C₄- to C₂₀-alkylcycloalkyl, aryl, C₇- to C₂₀-alkylaryl, C₇- to C₂₀-aralkyl, or aryl or C₇- to C₂₀-aralkyl which is mono- to trisubstituted by C₁- to C₈-alkyl, at from 0 to 180°C and from 0.01 to 10 bar, which comprises carrying out the reaction in the presence of a base and of a quaternary ammonium or phosphonium salt.

2. A process for preparing N-alkenylazoles as claimed in claim 1, wherein R³ and R⁵ are hydrogen or C₁- to C₂₀-alkyl.

3. A process for preparing N-alkenylazoles as claimed in claim 1 or 2, wherein R³ and R⁵ are hydrogen or C₁- to C₁₂-alkyl.

4. A process for preparing N-alkenylazoles as claimed in claims 1 to 3, wherein R³ and R⁵ are hydrogen.

5. A process for preparing N-alkenylazoles as claimed in claims 1 to 4, wherein R¹ and R² are hydrogen.

6. A process for preparing N-alkenylazoles as claimed in claims 1 to 5, wherein R⁷ is hydrogen, C₁- to C₂₀-alkyl, C₃- to C₈-cycloalkyl, aryl, C₇- to C₂₀-alkylaryl or C₇- to C₂₀-aralkyl.

7. A process for preparing N-alkenylazoles as claimed in claims 1 to 6, wherein the reaction is carried out at from 40 to 150°C.

8. A process for preparing N-alkenylazoles as claimed in claims 1 to 7, wherein the reaction is carried out at from 50 to 120°C.

9. A process for preparing N-alkenylazoles as claimed in claims 1 to 8, wherein the reaction is carried out at from 0.1 to 2 bar.

10. A process for preparing N-alkenylazoles as claimed in claims 1 to 9, wherein the reaction is carried out at atmospheric pressure.

## Revendications

1. Procédé pour la préparation de N-alcényl-azoles de formule générale I 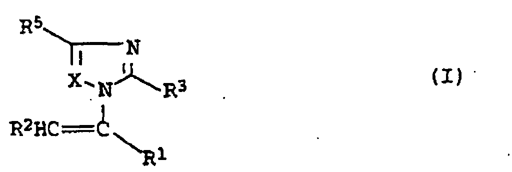 dans laquelle
X représente un groupe CH ou un atome d'azote,
R¹, R² représentent un atome d'hydrogène ou un groupe alkyle en C₁ à C₈,
R³, R⁵ représentent un atome d'hydrogène, un groupe alkyle en C₁ à C₄₀, alcényle en C₂ à C₄₀, cycloalkyle en C₃ à C₂₀, alkyl-cycloalkyle en C₄ à C₂₀, cycloalkyl-alkyle en C₄ à C₂₀, aryle, alkylaryle en C₇ à C₂₀, aralkyle en C₇ à C₂₀, un groupe alkylaryle en C₇ à C₂₀, aralkyle en C₇ à C₂₀ ou aryle qui sont substitués, une à cinq fois, par un groupe alkyle en C₁ à C₈, alcoxy en C₁ à C₈ ou par un atome d'halogène,
au moyen d'une réaction des azoles de formule générale II 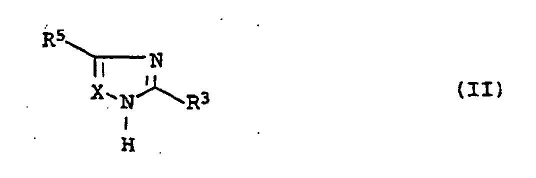 dans laquelle X, R³ et R⁵ prennent les significations susmentionnées, avec un ester alcénylique de l'acide carboxylique, de formule générale III 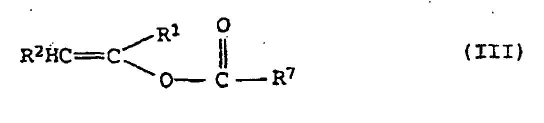 dans laquelle R¹ et R² prennent les significations susmentionnées et R⁷ représente un atome d'hydrogène, un groupe alkyle en C₁ à C₄₀, cycloalkyle en C₃ à C₂₀, alkyl-cycloalkyle en C₄ à C₂₀, aryle, alkylaryle en C₇ à C₂₀, aralkyle en C₇ à C₂₀, un groupe aralkyle en C₇ à C₂₀ ou aryle qui sont substitués, une à trois fois, par un groupe alkyle en C₁ à C₈, à une température allant de 0°C à 180°C et sous une pression allant de 0,01 bar à 10 bars, **caractérisé en ce que** l'on réalise la réaction en présence d'une base et d'un sel d'ammonium ou de phosphonium quaternaire.

2. Procédé pour la préparation de N-alcényl-azoles selon la revendication 1, **caractérisé en ce que** R³ et R⁵ représentent un atome d'hydrogène ou un groupe alkyle en C₁ à C₂₀.

3. Procédé pour la préparation de N-alcényl-azoles selon les revendications 1 à 2, **caractérisé en ce que** R³ et R⁵ représentent un atome d'hydrogène ou un groupe alkyle en C₁ à C₁₂.

4. Procédé pour la préparation de N-alcényl-azoles selon les revendications 1 à 3, **caractérisé en ce que** R³ et R⁵ représentent un atome d'hydrogène.

5. Procédé pour la préparation de N-alcényl-azoles selon les revendications 1 à 4, **caractérisé en ce que** R¹ et R² représentent un atome d'hydrogène.

6. Procédé pour la préparation de N-alcényl-azoles selon les revendications 1 à 5, **caractérisé en ce que** R⁷ représente un atome d'hydrogène, un groupe alkyle en C₁ à C₂₀, cycloalkyle en C₃ à C₈, aryle, alkylaryle en C₇ à C₂₀ ou aralkyle en C₇ à C₂₀.

7. Procédé pour la préparation de N-alcényl-azoles selon les revendications 1 à 6, **caractérisé en ce que** l'on réalise la réaction à une température allant de 40°C à 150°C.

8. Procédé pour la préparation de N-alcényl-azoles selon les revendications 1 à 7, **caractérisé en ce que** l'on réalise la réaction à une température allant de 50°C à 120°C.

9. Procédé pour la préparation de N-alcényl-azoles selon les revendications 1 à 8, **caractérisé en ce que** l'on réalise la réaction sous une pression allant de 0,1 bar à 2 bars.

10. Procédé pour la préparation de N-alcényl-azoles selon les revendications 1 à 9, **caractérisé en ce que** l'on réalise la réaction sous pression atmosphérique.
